(19) Europäisches Patentamt
European Patent Office
Office européen des brevets

(11) **EP 1 669 755 B1**

(12) **EUROPÄISCHE PATENTSCHRIFT**

(45) Veröffentlichungstag und Bekanntmachung des Hinweises auf die Patenterteilung:
**07.02.2007 Patentblatt 2007/06**

(51) Int Cl.:
*G01N 33/15* (2006.01)   *G01N 22/04* (2006.01)
*G01G 17/00* (2006.01)

(21) Anmeldenummer: **04029055.3**

(22) Anmeldetag: **08.12.2004**

(54) **Verfahren und Vorrichtung zum Messen der Masse und/oder Feuchte des Inhalts von Kapseln**

Method and device for measuring mass and/or moisture of the content of capsules

Procédé et dispositif de mesure de la masse et/ou de l'humidité du contenu de capsules

(84) Benannte Vertragsstaaten:
**AT BE BG CH CY CZ DE DK EE ES FI FR GB GR HU IE IS IT LI LT LU MC NL PL PT RO SE SI SK TR**

(43) Veröffentlichungstag der Anmeldung:
**14.06.2006 Patentblatt 2006/24**

(73) Patentinhaber: **TEWS ELEKTRONIK Dipl.-Ing. Manfred Tews**
**22459 Hamburg (DE)**

(72) Erfinder:
• **Herrmann, Rainer**
**20253 Hamburg (DE)**
• **Schlemm, Udo, Dr.**
**20259 Hamburg (DE)**

(74) Vertreter: **Glawe, Delfs, Moll**
**Patent- und Rechtsanwälte,**
**Rothenbaumchaussee 58**
**20148 Hamburg (DE)**

(56) Entgegenhaltungen:
**EP-A- 0 889 321       EP-A- 1 467 191**
**EP-A- 1 484 586       WO-A-20/04005903**

## Beschreibung

Die Erfindung betrifft ein Verfahren zum Messen der Masse und/oder Feuchte des Inhalts von insbesondere medizinische, pharmazeutische, Vitamine und ähnliche Produkte enthaltenden Kapseln mit Hilfe von Mikrowellen, bei dem durch die Kapseln in einen Resonator bewirkte Verschiebung der Resonanzfrequenz und Verbreiterung der Resonanzkurve bestimmt und ausgewertet wird, und eine Vorrichtung zum Durchführen des Verfahrens.

Mit einem solchen Verfahren kann die Masse und/oder die Feuchte von portionierten Wirkstoffeinheiten, insbesondere auch Kapseln bestimmt werden (EP 1 467 191 A1). Ist das Gewicht der Kapselhülle bekannt, so ergibt sich durch Differenzbildung auch die Masse des Inhalts der Kapsel, der insbesondere der medizinische, pharmazeutische, Vitamine oder ähnliche Produkte enthält. Ist die Masse des Inhalts der Kapsel aber wesentlich geringer als die Masse der Kapselhülle, so führen ungleichmäßige Massen der Kapselhüllen zu erheblichen Fehlern bei der Bestimmung der Masse des In-halts. Eine beispielhafte Kapsel hat eine Masse von 50 mg während die Masse des aus Trägermaterial und Wirkstoff bestehenden Inhalts der Kapsel 5 mg beträgt. Bei diesem typischen Beispiel zeigt sich schon, dass wenn die Kapselmasse um +/-5 mg schwankt, dass eine Messung der Masse des Inhalts an fertig befüllten Kapseln nicht mehr möglich ist, selbst wenn die Gesamtgenauigkeit der Messung weniger als 0,5 mg beträgt.

Gerade bei kleinen Mengen von Inhalts- bzw. Wirkstoffen kommt es aber sehr genau darauf an, die in der Kapsel enthaltene Menge zu kennen. Zu diesem Zweck könnte man zum Beispiel die Kapsel vor und nach dem Befüllen wiegen. Neben _dem dazu nötigen Eingriff in die Befüllungsmaschine benötigt jedoch der mechanische Vorgang des Wiegens immer eine gewisse Zeit, sodass man, um eine genügend hohe Durchsatzgeschwindigkeit zu erhalten, viele Waagen parallel zueinander anordnen müsste. Ein weiteres Beispiel besteht in der Messung der Gesamtmasse der leeren und der befüllten Kapsel im Bereich der Befüllungsmaschine durch zwei Mikrowellen-Messsysteme in entsprechender Anordnung (EP 1467191 A1).

Die Aufgabe der Erfindung besteht in der Schaffung eines Verfahrens zur Kontrollmessung fertig befüllter Kapseln, mit dem Masse und/oder Feuchte des Kapselinhalts schnell und zuverlässig gemessen werden kann.

Die erfindungsgemäße Lösung besteht darin, dass nicht nur die Gesamtmasse und/oder Gesamtfeuchte der gefüllten Kapsel bestimmt wird, sondern mit weiteren Messungen der Kapsel die unterschiedliche Wechselwirkung der einzelnen Teile der Kapsel mit elektromagnetischen Feldern bestimmt und ausgewertet wird.

Diese weiteren Messungen sind lokalisiert. Sie messen also nicht die Gesamtwerte der gefüllten Kapsel, sondern Werte an bestimmten Stellen.

So kann die Feuchte der Kapselhülle mit Hilfe elektromagnetischer Felder bestimmt werden. Diese Messung reicht in den meisten Fällen aus, da die Feuchte des Kapselinhalts entweder null beträgt oder aber dieser Kapselinhalt immer die gleiche Feuchte hat. Aus den Messwerten kann dann die Masse des Inhalts der Kapsel bestimmt werden.

Die Feuchte der Kapselhülle kann bei einer Ausführungsform mit Infrarotstrahlung bestimmt werden. Da dieses nur eine bestimmte Eindringtiefe hat, wird der Messwert nur durch die Feuchte der Kapselhülle, nicht aber durch die Feuchte des Kapselinhalts beeinflusst. Bei Feuchte null des Inhalts oder bekanntem konstanten Feuchtewert kann dann wieder die Masse des Kapselinhalts bestimmt werden.

Bei einer alternativen Ausführungsform wird die Feuchte der Kapselhülle mit Hilfe von Mikrowellen in einem Resonator bestimmt, der ein so scharf begrenztes Messvolumen ausweist, dass damit nur ein Teil der Kapselhülle ohne Inhalt erfassbar ist. Nur die Kapselhülle beeinflusst dann den Messwert. Ist so der Feuchtewert der Kapselhülle bestimmt, so kann bei Feuchte null oder bekannter konstanter Feuchte des Inhalts wieder die Masse des Inhalts bestimmt werden.

In vielen Fällen, wenn nicht in der überwiegenden Zahl der Fälle füllt der Inhalt die Kapsel nicht vollständig aus. In diesem Falle ist es mit dem erfindungsgemäßen Verfahren möglich, die Masse des Inhalts zu bestimmen, auch wenn dessen Feuchte variiert und nicht bekannt ist. Dies kann bei einer Ausführungsform dadurch erreicht werden, dass der Inhalt der Kapsel durch Beschleunigung oder Schwerkraft auf eine Seite des Kapselhohlraumes verfrachtet wird und die Feuchte der Kapsel mit Hilfe von Mikrowellen als Funktion der Messstelle über die Länge der Kapsel bestimmt wird. Es wird dabei also nicht nur die Gesamtmasse und die Gesamtfeuchte der gefüllten Kapsel in einem homogenen Messfeld bestimmt, sondern die Feuchte als Funktion der Messstelle über die Länge der Kapsel. Hierfür sind Profilsensoren bekannt, die eine Messung über ein verhältnismäßig kleines Messvolumen ermöglichen (EP 0 889 321 A1).

Auf der Seite der Kapsel, auf der sich der Inhalt befindet, wird das Mikrowellensignal durch die Feuchte der Kapselhülle und der Feuchte des Kapselinhalts beeinflusst, während auf der anderen Seite, an der sich kein Inhalt befindet, das Mikrowellensignal nur durch die Feuchte der Kapselhülle beeinflusst wird. In diesem Falle ist es möglich, getrennt voneinander Feuchte von Kapsel und Inhalt und andererseits nur der Kapselhülle zu bestimmen. Daraus kann wieder die Masse des Inhalts bestimmt werden.

Diese Messung kann dadurch durchgeführt werden, dass ein erster Resonator mit über die Kapselabmessungen homogenem Messfeld zur Bestimmung der Gesamtmasse/feuchte der Kapsel verwendet wird und ein zweiter Resonator mit einem engen Messfeld (Profilsensor) für die Bestimmung der ortsabhängigen Masse/Feuchtewerte verwendet wird. Dabei ist dann nur sicherzustellen, dass die Messungen in den beiden Resonatoren der selben Kapsel zugeordnet werden. Auf die Reihenfolge kommt es dabei nicht an. Es kann zuerst die Messung im homogenen Messfeld und dann im Profilsensor oder umgekehrt erfolgen.

Die Messungen können aber auch nur mit einem Resonator durchgeführt werden, der ein enges Messfeld ausweist (Profilsensor). In diesem Falle wird die Gesamtmasse/feuchte durch Integration der einzelnen über die Länge der Kapsel ermittelten Werte ermittelt. Dazu ist es erforderlich, dass die Kapseln mit der gleichen Geschwindigkeit durch das Messvolumen bewegt werden.

Bei einer anderen Ausführungsform mit dem ähnlichen Aufbau wird die Kapsel nicht mit der gleichen Geschwindigkeit durch das Messvolumen bewegt, es wird aber die Geschwindigkeit jeweils gemessen, mit der die Kapsel durch das Messfeld bewegt.

Die Messungen werden vorteilhafterweise bei einer Mikrowellenfrequenz von 1 bis 50 GHz insbesondere 1 bis 5 GHz durchgeführt.

Die Erfindung betrifft auch eine Vorrichtung zur Durchführung des Verfahren.

Die Erfindung wird im Folgenden anhand von vorteilhaften Ausführungsformen in Bezugnahme auf die beiden beigefügten Zeichnungen beschrieben. Es zeigen:

Fig. 1    das Schema einer Kapsel mit Ihrem Inhalt;

Fig. 2    den gemessenen Mikrowellen-Feuchtewert und die Resonanzfrequenzverschiebung über die Längenausdehnung der Kapsel der Fig. 1;

Fig. 3    eine erste Messvorrichtung, mit der das erfindungsgemäße Verfahren durchgeführt werden kann;

Fig. 4a    einen Mikrowellensensor mit einem Messfeld, der für das Verfahren der Erfindung verwendet werden kann;

Fig. 4b    die Mikrowellenfeldstärke über die Längenausdehnung des Resonators der Fig. 4a;

Fig. 5    eine andere Vorrichtung, mit der das Verfahren der Erfindung ausgeübt werden kann;

Fig. 6    eine noch andere Vorrichtung, mit der das Verfahren der Erfindung ausgeführt werden kann;

Fig. 7    eine weitere Ausführungsform, mit der das Verfahren der Erfindung durchgeführt werden kann;

Fig. 8    eine grafische Darstellung der mit dem erfindungsgemäßen Verfahren bestimmten Masse des Inhalts in Abhängigkeit von der mit einer Waage bestimmten Füllmasse für unterschiedliche Kapselgrößen.

Beim Mikrowellen-Resonanzverfahren werden bei jeder Messung zwei Resonanzparameter gemessen. Die erste der gemessenen Größen ist die Verschiebung der Resonanzfrequenz A in Hz:

$$A = f_0 - f_m \qquad\qquad (1)$$

mit $f_0$: Resonanzfrequenz des leeren Resonators in Hz
$f_m$: Resonanzfrequenz des gefüllten Resonators in Hz

Die zweite gemessene Größe ist die Vergrößerung der Halbwertsbreite der Resonanz B in Hz:

$$B = w_m - w_0 \qquad\qquad (2)$$

mit $w_0$: Halbwertbreite der Resonanz des leeren Resonators in Hz
$w_m$: Halbwertsbreite der Resonanz des gefüllten Resonators in Hz

Da beide Parameter A und B in gleicher Weise masseabhängig sind, ist der Quotient der beiden Größen masseunabhängig. Der masseunabhängige Mikrowellen-Feuchtewert F ergibt sich folgendermaßen aus diesem Quotienten:

$$F=arctan\ (B/A) \qquad\qquad (3)$$

Durch die Messung der zwei Parameter A und B kann neben der Feuchte des Messobjektes unter Vorraussetzung, dass das Messfeld, in dem sich das Messgut befindet, homogen ist, auch die Masse des Messgutes bestimmt werden.

Bei der Messung des Inhalts befüllter Kapseln sind die folgenden Kenngrößen unbekannt:

1. Die Masse der Kapselhülle $m_k$
2. Die Masse des Inhalts $m_i$
3. Die Masse des Wassers in der Kapselhülle $m_{wk}$
4. Die Masse des Wassers in dem Kapselinhalt $m_{wi}$

In der Praxis ist die Masse des Wassers in dem Kapselinhalt $m_{wi}$ häufig gleich null bzw. konstant, muss also bei vielen Messanwendungen nicht bestimmt werden. Da also 3 bzw. 4 Kenngrößen unbekannt sind, müssen 3 bzw. 4 unabhängige Größen gemessen werden, um die Masse des Kapselinhaltes $m_i$ bestimmen zu können. Verschiedene Möglichkeiten zur Realisierung dieser Messung werden in folgenden erläutert.

**1. Kombination eines integrierenden Mikrowellenresonators mit einem Resonator zur Profilmessung**

Unter einem integrierten Mikrowellenresonator versteht man einen Resonator, der ein Messfeld konstanter Feldstärke aufweist, das mindestens so groß ist wie das zu messende Objekt. Das Messobjekt muss sich bei der Messung komplett in diesem homogenen Messfeld befinden. Die hierbei gemessenen Resonanzgrößen $A_{int}$ und $B_{int}$ sind hierbei abhängig von Masse- und Feuchteanteilen von Kapselhülle und Inhalt:

$$A_{int}=a_1 \cdot m_k + a_2 \cdot m_i + a_3 \cdot m_{wk} + a_4 \cdot m_{wi} \qquad (4)$$

$$B_{int}=b_1 \cdot m_k + b_2 \cdot m_i + b_3 \cdot m_{wk} + b_4 \cdot m_{wi} \qquad (5)$$

Bezeichnungen:
$A_{int}$: Resonanzfrequenzverschiebung durch gesamte Kapsel
$B_{int}$: Verbreiterung der Resonanzkurve durch gesamte Kapsel
$m_k$: Masse der Kapselhülle
$m_i$: Masse des Kapselinhaltes
$m_{wk}$: Masse des Wassers in der Kapselhülle
$m_{wi}$: Masse des Wassers in dem Kapselinhalt
$a_i$, $b_i$: Koeffizienten

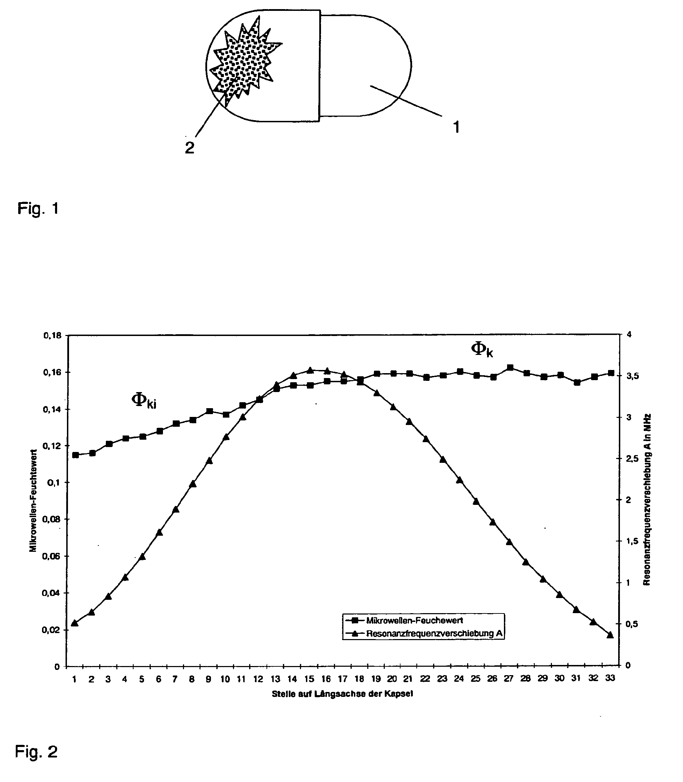

Ein Mikrowellenresonator zu Profilmessung ermöglicht eine Messung von Profilen von Feuchte- und Masse mit einer Ortsauflösung, die wesentlich kleiner als die Längsausdehnung des Messgutes ist, in diesem Fall typischerweise $\leq$ 3mm. Ein solcher Profilresonator wurde von der Fa. TEWS Elektronik bereits unter der Nummer EP 0 889 321 A1 zum Patent angemeldet. Bei der Messung partiell gefüllter medizinischer Kapseln ist bei der Kapselführung dafür zu sorgen, dass sich der Kapselinhalt 2 durch Trägheits- oder Gravitationskräfte an einem Ende der Kapsel 1 befindet, wie dies in Fig. 1 gezeigt ist. Dies kann durch starke Beschleunigung oder senkrechte Positionierung erreicht werden. Für diesen Fall zeigt Fig. 2 beispielhaft die gemessenen Profile der Verschiebung der Resonanzfrequenz A durch Beziehung (1) und des Mikrowellen-Feuchtewertes F nach Beziehung (3) (Kapselgröße 5, Füllung: 94mg Maismehl). Aus dem linken Bereich des Profils des Mikrowellen-Feüchtewertes F kann der gemeinsame Mikrowellen-Feuchtewert von Kapselinhalt und Kapselhülle $F_{ki}$ bestimmt werden. Im rechten Profilbereich ist die Kapselhülle leer, man kann deshalb hier den Mikrowellen-Feuchtewert der leeren Kapsel $F_k$ bestimmen.

Für diese Werte gelten die folgenden Beziehungen:

$$F_k = c_1 \cdot m_{wk} / (m_{wk} + m_k) + c_2 \qquad (6)$$

$$F_{ki} = F_k + d_1 \cdot m_{wi} / (m_{wi} + m_i) + d_2 \qquad (7)$$

Bezeichnungen:

$F_{ki}$: Mikrowellen-Feuchtewert von Kapselinhalt und Kapselhülle
$F_k$: Mikrowellen-Feuchtewert der leeren Kapsel
$m_k$: Masse der Kapselhülle
$m_i$: Masse des Kapselinhaltes
$m_{wk}$: Masse des Wassers in der Kapselhülle
$m_{wi}$: Masse des Wassers in dem Kapselinhalt
$c_i$, $d_i$: Koeffizienten

Die Koeffizienten $a_i$, $b_i$, $c_i$ und $d_i$ können durch Kalibrationsmessungen bestimmt werden. Es existiert damit ein Satz von vier gemessenen Größen, aus denen die oben genannten vier unbekannten Größen bestimmt werden können und somit auch die gesuchte Masse des Kapselinhaltes $m_i$. Hierzu gilt es, das aus Beziehungen (4) - (7) bestehende lineare Gleichungssystem zu lösen.

Für den häufig in der pharmazeutischen Praxis vorkommenden Fall, dass der Kapselinhalt keine Feuchte aufnimmt, oder diese nicht variiert, ist nur die Bestimmung dreier Messgrößen nötig: der Resonanzfrequenzverschiebung und der Verbreiterung der Resonanzkurve durch die gesamte Kapsel, d.h. $A_{int}$ und $B_{int}$, sowie des Mikrowellen-Feuchtewertes der leeren Kapsel $F_k$. Es resultiert ein reduziertes Gleichungssystem zur Bestimmung der Masse des Kapselinhaltes $m_i$:

$$A_{int} = a_1 \cdot m_k + a_2 \cdot m_i + a_3 \cdot m_{wk} \qquad (8)$$

$$B_{int} = b_1 \cdot m_k + b_2 \cdot m_i + b_3 \cdot m_{wk} \qquad (9)$$

$$F_k = c_1 \cdot m_{wk} / (m_{wk} + m_k) + c_2 \qquad (10)$$

Bezeichnungen:

$A_{int}$: Resonanzfrequenzverschiebung durch gesamte Kapsel
$B_{int}$: Verbreiterung der Resonanzkurve durch gesamte Kapsel
$F_k$: Mikrowellen-Feuchtewert der leeren Kapsel
$m_k$: Masse der Kapselhülle
$m_i$: Masse des Kapselinhaltes
$M_{wk}$: Masse des Wassers in der Kapselhülle
$a_i$, $b_i$, $c_i$: Koeffizienten

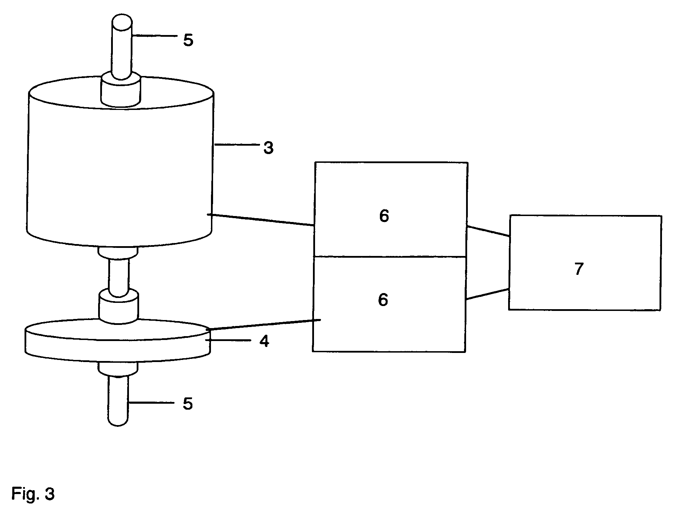

Eine Vorrichtung zur Durchführung dieses Verfahrens ist in Fig. 3 gezeigt. Sie weist einen Mikrowellenresonator 3 mit einem mindestens über das Kapselvolumen homogenen Messfeld und einen Profilsensor 4 auf, das ein sehr enges Messfeld in der Größenordnung von 3 mm in der Richtung, in der sich die Kapseln bewegen, aufweist. Durch die Resonatoren 3, 4 führt ein Rohr 5, durch das die zu vermessenden Kapseln hindurchgeleitet werden. Die Signale werden in Mikrowellen-und Auswertungseinheiten 6 ausgewertet und die Messergebnisse durch eine Einheit 7 berechnet und angezeigt, ausgedruckt oder auf andere Weise dokumentiert.

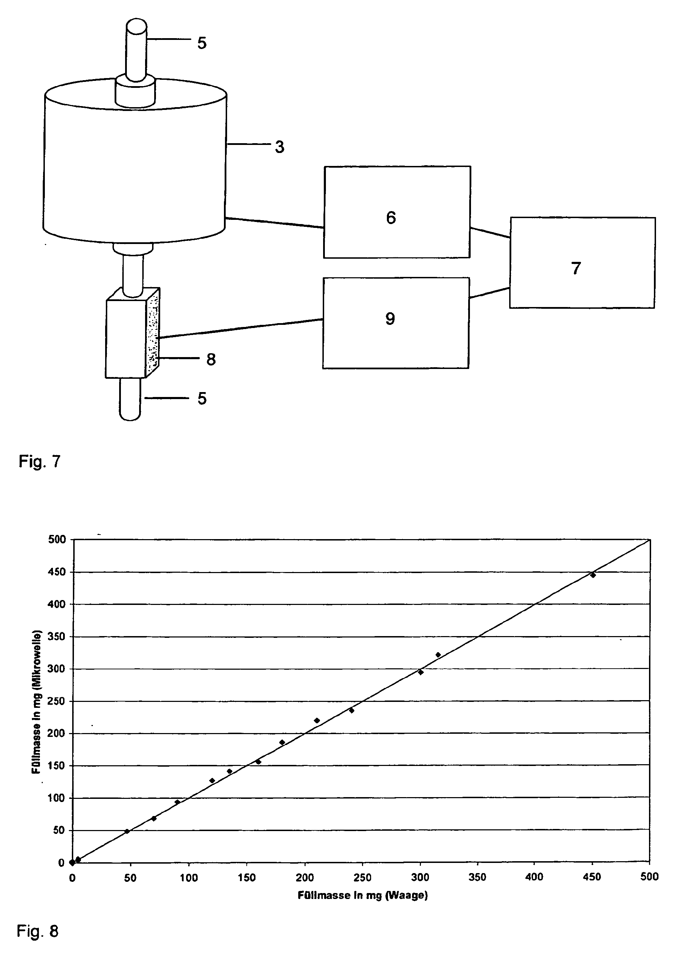

Fig. 8 zeigt Messergebnisse, die mit einer Kombination eines intergrierenden Mikrowellenresonators mit einem Resonator zur Profilmessung an teilweise gefüllten Hartgelantinekapseln erzielt wurden, wobei die unterschiedlichsten Kapselgrößen von Kgr.0 - Kgr.5 und Füllmengen von 0 - 450 mg verwendet wurden. Wie man sieht, ist das erfindungsgemäße Verfahren der Bestimmung der Masse des Inhalts also unabhängig von der Kapselgröße.

Im Falle vollständig gefüllter Kapseln kann die Feuchte der Kapselhülle nicht durch eine Profilmessung bestimmt werden, und somit versagt die Bestimmung der Masse des Inhaltes. Da jedoch bei vollständiger Füllung gilt: $m_k < m_i$ und die Schwankungen von $m_k$ deutlich kleiner als $m_i$ sind, kann die Bestimmung der Masse des Kapselinhaltes i.a. mit ausreichender Genauigkeit über die Gesamtmasse der Kapsel unter Berücksichtigung eines Durchschnittswertes der Massen der Kapselhüllen bestimmt werden. Hierfür reicht die Messung mit einem intergrierenden Mikrowellenresonators aus, vergl. TEWS Patentanmeldung "Verfahren und Vorrichtung zum Bestimmen der Masse von portionierten Wirkstoffen" EP 1 467 191 A1.

**2. Kombination eines intergierenden Mirkowellenresonators mit einem Resonator zur Messung der Oberflächenfeuchte**

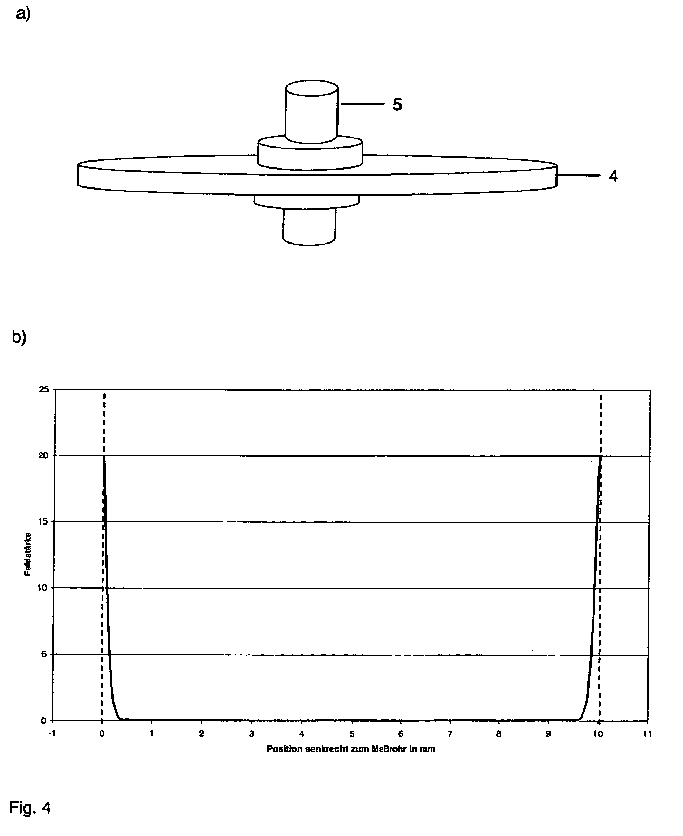

Ein Mikrowellenresonator zur Messung der Oberflächenfeuchte hat ein Messfeld, das nur an der Oberfläche des Messobjekts eine zur Messung geeignete Feldstärke aufweist, d.h. die Eigenschaften des Messobjektes können nur an seiner Oberfläche gemessen werden. Ein Beispiel für einen solchen Resonator 4 ist in Fig. 4 dargestellt. Durch einen solchen Resonator kann man den Mikrowellen-Feuchtewert der leeren Kapsel $F_k$ messen. Durch den intergrierenden Mikrowellenresonator werden auch in diesem Fall die Resonanzfrequenzverschiebung und die Verbreiterung der Resonanzkurve durch die gesamte Kapsel, d.h. $A_{int}$ und $B_{int}$ gemessen. Eine Messung an Kapseln mit einem Inhalt, dessen Feuchte variiert, ist in diesem Fall nicht möglich. Zur Auswertung der Messungen ist das Gleichungssystem (8) - (10) zu lösen. Fig. 3 zeigt schematisch den Aufbau eines Meßsystems, das für diesen Fall verwendet werden kann. Diese Messungen können auch durchgeführt werden, wenn die Kapseln vollständig gefüllt sind.

**3. Kombination eines integrierenden Mikrowellenresonators mit einer Feuchtemessung der Kapselhüllen**

Auch mit einem Infrarot-Feuchtemesssystem ist es möglich, bei allen Nachteilen der Infrarot-Feuchtemesstechnik, die Feuchte der Kapselhüllen wie beim vorher erwähnten Verfahren unabhängig von der des Füllgutes zu messen, da es sich hierbei um eine Oberflächenmessung handelt. Aus einer solchen Messung würde ein Feuchte-Messwert resultieren, der dem oben beschriebenen Mikrowellen-Feuchtewert der leeren Kapsel $F_k$ entspricht. Durch den intergrierenden Mikrowellenresonator werden auch in diesem Fall die Resonanzfrequenzverschiebung und die Verbreiterung der Resonanzkurve durch die gesamte Kapsel, d.h. $A_{int}$ und $B_{int}$ gemessen. Eine Messung an Kapseln mit einem Inhalt, dessen Feuchte variiert, ist aber in diesem Fall nicht möglich. Zur Auswertung der Messungen ist das Gleichungssystem (8) - (10) zu lösen.

Fig. 7 zeigt schematisch den Aufbau des Messsystems. Die Kapseln durchlaufen in der Röhre 5 zunächst einen integrierenden Sensor 3, mit dem mit Hilfe der Mikrowellen- und Auswerteeinheit 6 die Gesamtmasse und die Gesamtfeuchte der Kapseln bestimmt wird. Mit einem Infrarotstrahler und - detektor 8 wird mit einer Auswerteelektronik 9 die Feuchte der Kapselhülle bestimmt. Das Ergebnis wird wiederum in der Einheit 7 berechnet und angezeigt.

**4. Kombination eines Mirkowellenresonators zur Profilmessung mit einer Einrichtung zur Geschwindigkeitsmessung der Kapseln**

Bei Verwendung eines einfachen Mikrowellen-Profilresonators 4 gibt es ebenfalls die Möglichkeit, vier Messwerte zu erfassen, um die oben genannten vier unbekannten Größen bestimmen zu können. In diesem Fall müssen die Resonanzfrequenzverschiebung und die Verbreiterung der Resonanzkurve durch Integration der Mikrowellensignale über die gesamte Kapsel bestimmt werden. Da diese Integrale geschwindigkeitsabhängig sind, kann durch eine geeignete Messmethode die Kapselgeschwindigkeit bestimmt und bei der Integralbidung berücksichtigt werden (z.B. optische Doppler-Radar-Methode). Die Meßwerte $A_{int}$ und $B_{int}$ werden in diesem Fall also folgendermaßen bestimmt:

$$A_{\text{int}} = \int_0^T A \cdot V \, dt \qquad (11)$$

$$B_{int} = \int_0^T B \cdot V dt \qquad\qquad (12)$$

Bezeichnungen:
$A_{int}$: Resonanzfrequenzverschiebung durch gesamte Kapsel
$B_{int}$: Verbreiterung der Resonanzkurve durch gesamte Kapsel
A: lokale Resonanzfrequenzverschiebung
B: lokale Verbreiterung der Resonanzkurve
V: Kapselgeschwindigkeit

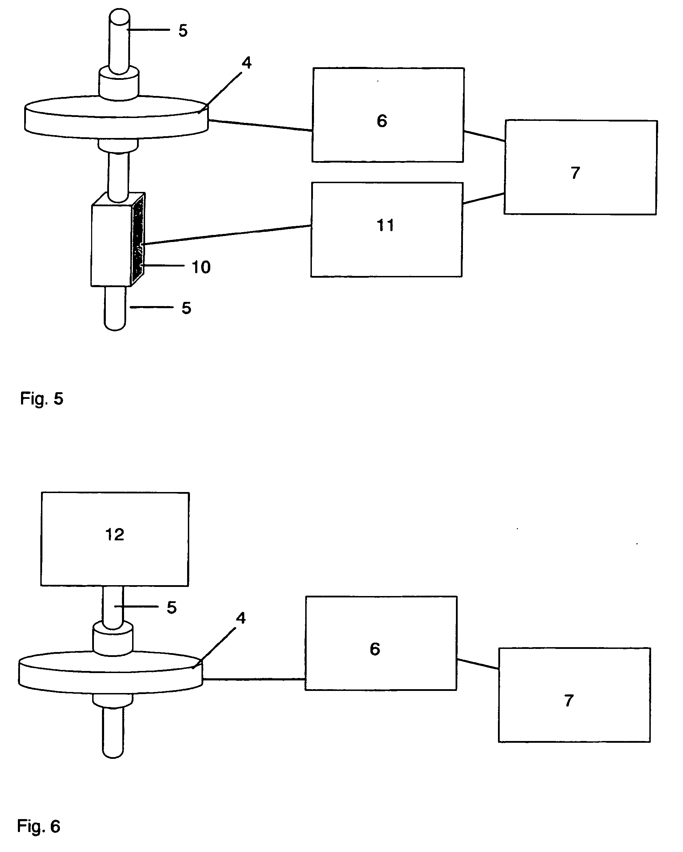

Die Integration erfolgt hierbei über die gesamte Zeit, während der sich die Kapsel im Messfeld befindet, d.h. von Feldeintritt bei t=0, bis Feldaustritt bei t=T. Neben Bestimmung von $A_{int}$ und $B_{int}$ nach Beziehungen (11) und (12) können aus dem Profil die Mikrowellen-Feuchtewerte $F_{ki}$ und $F_k$ gewonnen werden (siehe Fig. 2). Die Berechnung des Kapselinhaltes erfolgt wiederum über Lösung des Gleichungssystems (4) - (7). Bei konstanter Feuchte des Kapselinhaltes ist auch in diesem Fall das reduzierte Gleichungssystem (8) - (10) zu lösen. Auch in diesem Fall ist die Messung der Masse des Inhaltes $m_i$ nur bei teilweise gefüllten Kapseln möglich. Eine schematische Skizze des Messsystems zeigt Fig. 6. Die Kapseln werden wiederum durch die Röhre 5 durch einen Profilsensor 4 bewegt. Dies kann z. B. durch eine Luftströmung geschehen. Mit einer Messeinrichtung 10 wird die Geschwindigkeit der Kapseln bestimmt und bei 11 ausgewertet. Die Anzeige erfolgt nach Auswertung und Intergierung des Mikrowellensignals in der Einheit 6 wiederum in der Einheit 7.

**5. Mikrowellenresonator zur Profilmessung bei Führung durch den Resonator mit konstanter Geschwindigkeit**

Die Verwendung eines einfachen Mikrowellen-Profilresonators ohne eine kombinierte Geschwindigkeitsmessung der Kapseln ist möglich, wenn sichergestellt werden kann, dass die Kapseln beim Transport durch den Resonator immer die selbe Geschwindigkeit haben. In diesem Fall braucht die Geschwindigkeit bei der Integralbildung zur Bestimmung von $A_{int}$ und $B_{int}$ nicht berücksichtigt zu werden:

$$A_{int} = \int_0^T A dt \qquad\qquad (13)$$

$$B_{int} = \int_0^T B dt \qquad\qquad (14)$$

Bezeichnungen:

$A_{int}$: Resonanzfrequenz durch gesamte Kapsel
Bint: Verbreiterung der Resonanzkurve durch gesamte Kapsel
A: lokale Resonanzfrequenzverschiebung
B: lokale Verbreiterung der Resonanzkurve

Die Integration erfolgt hierbei über die gesamte Zeit, während der sich die Kapsel im Messfeld befindet, d.h. von Feldeintritt bei t=0, bis Feldaustritt bei t=T.
Neben Bestimmung von $A_{int}$ und Bint nach Beziehungen (11) und (12) können aus dem Profil die Mikrowellen-Feuchtewerte $F_{ki}$ und $F_k$ gewonnen werden(siehe Fig. 2). Die Berechnung des Kapselinhaltes erfolgt wiederum über Lösung des Gleichungssystems (4) - (7). Bei konstanter Feuchte des Kapselinhaltes ist auch in diesem Fall das reduzierte Gleichungssystem (8) - (10) zu lösen. Auch in diesem Fall ist die Messung der Masse des Inhaltes $m_i$ nur bei teilweise gefüllten Kapseln möglich.

In Fig. 6 ist schematisch eine Vorrichtung zur Ausführung des Verfahrens gezeigt. Durch eine bei 12 angeordnete Einheit wird dafür gesorgt, das die Kapseln mit gleichmäßiger und bekannter Geschwindigkeit durch die Röhre 5 gelangen. Sie werden dann durch den Profilsenor 4 und die Mikrowellen- und Auswerteelektronik 6 vermessen. Das Ergebnis wird wiederum in der Einheit 7 berechnet und angezeigt.

**Patentansprüche**

1. Verfahren zum Messen der Masse und/oder der Feuchte des Inhalts (2) von insbesondere medizinische, pharmazeutische, Vitamine und ähnliche Produkte enthaltenden unvollständig gefüllten Kapseln (1) mit Hilfe von Mikrowellen, bei dem die durch die Kapseln (1) in mindestens zwei Resonatoren (3, 4) bewirkte Verschiebung der Resonanzfrequenz (A) und Verbreiterung der Resonanzkurve (B) bestimmt und ausgewertet wird, wobei ein erster Resonator (3) mit über die Kapselabmessungen homogenem Messfeld zur Bestimmung der Gesamtmasse/feuchte der Kapsel (1) verwendet wird, **dadurch gekennzeichnet, dass** der Inhalt (2) der Kapsel (1) durch Beschleunigung oder Schwerkraft auf eine Seite des Kapselhohlraums verfrachtet wird und dass ein zweiter Resonator (4) mit einem engen Messfeld für die Bestimmung ortsabhängiger Profile von Masse/Feuchtewerten verwendet wird.

2. Verfahren zum Messen der Masse und/oder der Feuchte des Inhalts (2) von insbesondere medizinische, pharmazeutische, Vitamine und ähnliche Produkte enthaltenden Kapseln (1) mit Hilfe von Mikrowellen, bei dem die durch die Kapseln (1) in mindestens zwei Resonatoren (3, 4) bewirkte Verschiebung der Resonanzfrequenz (A) und Verbreiterung der Resonanzkurve (B) bestimmt und ausgewertet wird, wobei ein erster Resonator (3) mit über die Kapselabmessungen homogenem Messfeld zur Bestimmung der Gesamtmasse/feuchte der Kapsel (1) verwendet wird, **dadurch gekennzeichnet, dass** ein zweiter Resonator (4) verwendet wird, der ein so scharf begrenztes Messvolumen aufweist, dass damit nur die Kapselhülle erfassbar ist.

3. Verfahren zum Messen der Masse und/oder der Feuchte des Inhalts (2) von insbesondere medizinische, pharmazeutische, Vitamine und ähnliche Produkte enthaltenden Kapseln (1) mit Hilfe von Mikrowellen, bei dem die durch die Kapseln (1) in mindestens einem Resonator (3, 4) bewirkte Verschiebung der Resonanzfrequenz (A) und Verbreiterung der Resonanzkurve (B) bestimmt und ausgewertet wird, wobei ein erster Resonator (3) mit über die Kapselabmessungen homogenem Messfeld zur Bestimmung der Gesamtmasse/feuchte der Kapsel (1) verwendet wird, **dadurch gekennzeichnet, dass** die Feuchte der Kapselhülle mit Hilfe von Infrarotstrahlung bestimmt wird.

4. Verfahren zum Messen der Masse und/oder der Feuchte des Inhalts (2) von insbesondere medizinische, pharmazeutische, Vitamine und ähnliche Produkte enthaltenden unvollständig gefüllten Kapseln (1) mit Hilfe von Mikrowellen, bei dem die durch die Kapseln (1) in einem Resonator (4) bewirkte Verschiebung der Resonanzfrequenz (A) und Verbreiterung der Resonanzkurve (B) bestimmt und ausgewertet wird, wobei ein Resonator (4) mit engem Messfeld für die Bestimmung ortsabhängiger Profile von Masse/Feuchtenwerten verwendet wird, **dadurch gekennzeichnet, dass** der Inhalt (2) der Kapsel (1) durch Beschleunigung oder Schwerkraft auf eine Seite des Kapselhohlraums verfrachtet wird und, dass die Geschwindigkeit gemessen wird (10), mit der die Kapsel (1) durch das Messfeld bewegt wird und dass die Gesamtmasse/feuchte der Kapsel durch Integration bestimmt wird.

5. Verfahren zum Messen der Masse und/oder der Feuchte des Inhalts (2) von insbesondere medizinische, pharmazeutische, Vitamine und ähnliche Produkte enthaltenden unvollständig gefüllten Kapseln (1) mit Hilfe von Mikrowellen, bei dem die durch die Kapseln (1) in einem Resonator (4) bewirkte Verschiebung der Resonanzfrequenz (A) und Verbreiterung der Resonanzkurve (B) bestimmt und ausgewertet wird, wobei ein Resonator (4) mit engem Messfeld für die Bestimmung ortsabhängiger Profile von Masse/Feuchtenwerten verwendet wird, **dadurch gekennzeichnet, dass** der Inhalt (2) der Kapsel (1) durch Beschleunigung oder Schwerkraft auf eine Seite des Kapselhohlraums verfrachtet wird und dass alle Kapseln (1) mit der gleichen Geschwindigkeit durch das Messvolumen bewegt werden (12) und die Gesamtmasse/feuchte der Kapseln (1) durch Integration bestimmt wird.

6. Verfahren nach einem der Ansprüche 1, 4 oder 5, **dadurch gekennzeichnet, dass** die Feuchte der Kapsel (1) mit Hilfe von Mikrowellen als Funktion der Messstelle über die Länge der Kapsel bestimmt wird.

7. Verfahren nach einem der Ansprüche 1 bis 6, **dadurch gekennzeichnet, dass** die Messungen bei einer Mikrowellenfrequenz von 1 bis 50 GHz, insbesondere 1 bis 5 GHz durchgeführt werden.

8. Vorrichtung zum Messen der Masse und/oder der Feuchte des Inhalts von insbesondere medizinische, pharmazeutische, Vitamine und ähnliche Produkte enthaltenden Kapseln (1) mit Hilfe von Mikrowellen, mit einem ersten Mikrowellenresonator (3) mit über die Ausdehnung der Kapsel (1) homogenem Messfeld, **dadurch gekennzeichnet, dass** sie Einrichtungen zum Verfrachten des Inhalts (2) der Kapsel (1) durch Beschleunigung oder Schwerkraft auf eine Seite des Kapselhohlraums und einen zweiten Mikrowellenresonator (4) zum Messen von Masse- und/oder Feuchteprofilen der Kapsel mit einem relativ zur Kapsellänge schmalen Messfeld aufweist.

9. Vorrichtung zum Messen der Masse und/oder der Feuchte des Inhalts von insbesondere medizinische, pharmazeutische, Vitamine und ähnliche Produkte enthaltenden Kapseln (1) mit Hilfe von Mikrowellen, mit einem ersten Mikrowellenresonator (3) mit über die Ausdehnung der Kapsel (1) homogenen Messfeld, **dadurch gekennzeichnet, dass** sie einen zweiten Mikrowellenresonator (4) umfasst, der ein so scharf begrenztes Messvolumen aufweist, dass damit nur die Kapselhülle erfassbar ist.

10. Vorrichtung zum Messen der Masse und/oder der Feuchte des Inhalts von insbesondere medizinische, pharmazeutische, Vitamine und ähnliche Produkte enthaltenden Kapseln (1) mit Hilfe von Mikrowellen, mit einem Mikrowellenresonator (3) mit über die Ausdehnung der Kapsel (1) homogenem Messfeld, **dadurch gekennzeichnet, dass** sie Infrarotmesseinrichtungen (8,9) zum Messen der Feuchte der Kapselhülle umfasst.

11. Vorrichtung zum Messen der Masse und/oder der Feuchte des Inhalts von insbesondere medizinische, pharmazeutische, Vitamine und ähnliche Produkte enthaltenden Kapseln (1) mit Hilfe von Mikrowellen, mit einem Mikrowellenresonator (4) mit einem relativ zur Kapsellänge schmalen Messfeld, **dadurch gekennzeichnet, dass** sie Einrichtungen zum Verfrachten des Inhalts (2) der Kapsel (1) durch Beschleunigung oder Schwerkraft auf eine Seite des Kapselhohlraums und eine Einrichtung (10, 11) zum Messen der Geschwindigkeit der Kapseln (1) aufweist.

12. Vorrichtung zum Messen der Masse und/oder der Feuchte des Inhalts von insbesondere medizinische, pharmazeutische, Vitamine und ähnliche Produkte enthaltenden Kapseln (1) mit Hilfe von Mikrowellen, mit einem Mikrowellenresonator (4) mit einem relativ zur Kapsellänge schmalen Messfeld, **dadurch gekennzeichnet, dass** sie Einrichtungen zum Verfrachten des Inhalts (2) der Kapsel (1) durch Beschleunigung oder Schwerkraft auf eine Seite des Kapselhohlraums und eine Einrichtung (12) zum Fördern der Kapseln (1) mit gleichbleibender Geschwindigkeit aufweist.

13. Vorrichtung nach einem der Ansprüche 8 bis 12, **dadurch gekennzeichnet, dass** sie mit Mikrowellenfrequenzen von 1 bis 50 GHz, insbesondere 1 bis 5 GHz betreibbar ist.

**Claims**

1. Method for measuring the mass and/or moisture of the contents (2) of incompletely filled capsules (1), containing, in particular, medicinal products, pharmaceutical products, vitamins and similar products, with the aid of microwaves, in which method the displacement of the resonant frequency (A) and broadening of the resonance curve (B) caused by the capsules (1) in at least two resonators (3, 4) is determined and evaluated, a first resonator (3) with a measuring field which is homogeneous over the capsule dimensions being used to determine the total mass/moisture of the capsule (1), **characterised in that** the contents (2) of the capsule (1) are transported to one side of the capsule cavity by acceleration or gravity, and **in that** a second resonator (4) with a narrow measuring field is used to determine location-dependent profiles of mass/moisture values.

2. Method for measuring the mass and/or moisture of the contents (2) of capsules (1), containing, in particular, medicinal products, pharmaceutical products, vitamins and similar products, with the aid of microwaves, in which method the displacement of the resonant frequency (A) and broadening of the resonance curve (B) caused by the capsules (1) in at least two resonators (3, 4) is determined and evaluated, a first resonator (3) with a measuring field which is homogeneous over the capsule dimensions being used to determine the total mass/moisture of the capsule (1), **characterised in that** a second resonator (4), having a measuring volume which is so severely limited that only the capsule casing can be detected therewith, is used.

3. Method for measuring the mass and/or moisture of the contents (2) of capsules (1), containing, in particular, medicinal products, pharmaceutical products, vitamins and similar products, with the aid of microwaves, in which method the displacement of the resonant frequency (A) and broadening of the resonance curve (B) caused by the capsules (1) in at least one resonator (3, 4) is determined and evaluated, a first resonator (3) with a measuring field which is homogeneous over the capsule dimensions being used to determine the total mass/moisture of the capsule (1), **characterised in that** the moisture of the capsule casing is determined with the aid of infrared radiation.

4. Method for measuring the mass and/or moisture of the contents (2) of incompletely filled capsules (1), containing, in particular, medicinal products, pharmaceutical products, vitamins and similar products, with the aid of microwaves, in which method the displacement of the resonant frequency (A) and broadening of the resonance curve (B) caused by the capsules (1) in a resonator (4) is determined and evaluated, a resonator (4) with a narrow measuring field being used to determine location-dependent profiles of mass/moisture values, **characterised in that** the contents (2) of the capsule (1) are transported to one side of the capsule cavity by acceleration or gravity, and **in that** the speed at which the capsule (1) is moved through the measuring field is measured (10), and **in that** the total mass/ moisture of the capsule is determined by integration.

5. Method for measuring the mass and/or moisture of the contents (2) of incompletely filled capsules (1), containing, in particular, medicinal products, pharmaceutical products, vitamins and similar products, with the aid of microwaves, in which method the displacement of the resonant frequency (A) and broadening of the resonance curve (B) caused by the capsules (1) in a resonator (4) is determined and evaluated, a resonator (4) with a narrow measuring field being used to determine location-dependent profiles of mass/moisture values, **characterised in that** the contents (2) of the capsule (1) are transported to one side of the capsule cavity by acceleration or gravity, and **in that** all the capsules (1) are moved at the same speed through the measuring volume (12) and the total mass/moisture of the capsules (1) is determined by integration.

6. Method according to one of Claims 1, 4 or 5, **characterised in that** the moisture of the capsule (1) is determined with the aid of microwaves as a function of the measuring point over the length of the capsule.

7. Method according to one of Claims 1 to 6, **characterised in that** the measurements are carried out at a microwave frequency of 1 to 50 GHz, in particular 1 to 5 GHz.

8. Apparatus for measuring the mass and/or moisture of the contents of capsules (1), containing, in particular, medicinal products, pharmaceutical products, vitamins and similar products, with the aid of microwaves, having a first microwave resonator (3) with a measuring field which is homogeneous over the extent of the capsule (1), **characterised in that** the apparatus has devices for transporting the contents (2) of the capsule (1) to one side of the capsule cavity by acceleration or gravity and a second microwave resonator (4) for measuring mass and/or moisture profiles of the capsule with a measuring field which is narrow relative to the capsule length.

9. Apparatus for measuring the mass and/or moisture of the contents of capsules (1), containing, in particular, medicinal products, pharmaceutical products, vitamins and similar products, with the aid of microwaves, having a first microwave resonator (3) with a measuring field which is homogeneous over the extent of the capsule (1), **characterised in that** the apparatus comprises a second microwave resonator (4) having a measuring volume which is so severely limited that only the capsule casing can be detected therewith.

10. Apparatus for measuring the mass and/or moisture of the contents of capsules (1), containing, in particular, medicinal products, pharmaceutical products, vitamins and similar products, with the aid of microwaves, having a microwave resonator (3) with a measuring field which is homogeneous over the extent of the capsule (1), **characterised in that** the apparatus comprises infrared measuring devices (8, 9) for measuring the moisture of the capsule casing.

11. Apparatus for measuring the mass and/or moisture of the contents of capsules (1), containing, in particular, medicinal products, pharmaceutical products, vitamins and similar products, with the aid of microwaves, having a microwave resonator (4) with a measuring field which is narrow relative to the capsule length, **characterised in that** the apparatus has devices for transporting the contents (2) of the capsule (1) to one side of the capsule cavity by acceleration or gravity and a device (10, 11) for measuring the speed of the capsules (1).

12. Apparatus for measuring the mass and/or moisture of the contents of capsules (1), containing, in particular, medicinal products, pharmaceutical products, vitamins and similar products, with the aid of microwaves, having a microwave resonator (4) with a measuring field which is narrow relative to the capsule length, **characterised in that** the apparatus has devices for transporting the contents (2) of the capsule (1) to one side of the capsule cavity by acceleration or gravity and a device (12) for conveying the capsules (1) at constant speed.

13. Apparatus according to one of Claims 8 to 12, **characterised in that** it is operable at microwave frequencies of 1 to 50 GHz, in particular 1 to 5 GHz.

**Revendications**

1. Procédé pour mesurer la masse et/ou l'humidité du contenu (2) de capsules (1) remplies de façon incomplète et contenant en particulier des produits médicaux, pharmaceutiques, à base de vitamines et des produits similaires à l'aide de micro-ondes, dans lequel le déplacement, provoqué par les capsules (1) dans au moins deux résonateurs (3, 4), de la fréquence de résonance (A) et l'élargissement de la courbe de résonance (B) sont déterminés et analysés, un premier résonateur (3) étant utilisé avec un champ de mesure homogène par rapport aux dimensions de capsule pour déterminer la masse/humidité globale de la capsule (1), **caractérisé en ce que** le contenu (2) de la capsule (1) est déplacé par l'accélération ou la force de gravité sur un côté de la cavité de capsule et **en ce qu'**un second résonateur (4) est utilisé avec un champ de mesure étroit pour la détermination de profils, dépendants du lieu, de valeurs de masse/humidité.

2. Procédé pour mesurer la masse et/ou l'humidité du contenu (2) de capsules (1) contenant en particulier des produits médicaux, pharmaceutiques, à base de vitamines et des produits similaires à l'aide de micro-ondes, dans lequel le déplacement, provoqué par les capsules (1) dans au moins deux résonateurs (3, 4), de la fréquence de résonance (A) et l'élargissement de la courbe de résonance (B) sont déterminés et analysés, un premier résonateur (3) étant utilisé avec un champ de mesure homogène par rapport aux dimensions de la capsule pour déterminer la masse/humidité globale de la capsule (1), **caractérisé en ce qu'**un second résonateur (4) est utilisé, lequel présente un volume de mesure limité si fortement que de ce fait seule l'enveloppe de capsule peut être détectée.

3. Procédé pour mesurer la masse et/ou l'humidité du contenu (2) de capsules (1) contenant en particulier des produits médicaux, pharmaceutiques, à base de vitamines et des produits similaires à l'aide de micro-ondes, dans lequel le déplacement, provoqué par les capsules (1) dans au moins un résonateur (3, 4), de la fréquence de résonance (A) et l'élargissement de la courbe de résonance (B) sont déterminés et analysés, un premier résonateur (3) étant utilisé avec un champ de mesure homogène par rapport aux dimensions de capsule pour déterminer la masse/humidité globale de la capsule (1), **caractérisé en ce que** l'humidité de l'enveloppe de capsule est déterminée à l'aide de rayonnement infrarouge.

4. Procédé pour mesurer la masse et/ou l'humidité du contenu (2) de capsules (1) remplies de façon incomplète et contenant en particulier des produits médicaux, pharmaceutiques à base de vitamines et des produits similaires à l'aide de micro-ondes, dans lequel le déplacement, provoqué par les capsules (1) dans un résonateur (4), de la fréquence de résonance (A) et l'élargissement de la courbe de résonance (B) sont déterminés et analysés, un résonateur (4) étant utilisé avec un champ de mesure resserré pour la détermination de profils, dépendants du lieu de valeurs de masse/humidité, **caractérisé en ce que** le contenu (2) de la capsule (1) est déplacé par l'accélération ou la force de gravité sur un côté de la cavité de capsule et **en ce que** la vitesse est mesurée (10), avec laquelle la capsule (1) est déplacée à travers le champ de mesure et **en ce que** la masse/humidité globale de la capsule est déterminée par intégration.

5. Procédé pour mesurer la masse et/ou l'humidité du contenu (2) de capsules (1) remplies de façon incomplète et contenant en particulier des produits médicaux, pharmaceutiques à base de vitamines et des produits similaires à l'aide de micro-ondes, dans lequel le déplacement, entraîné par les capsules (1) dans un résonateur (4), de la fréquence de résonance (A) et l'élargissement de la courbe de résonance (B) sont déterminés et analysés, un résonateur (4) étant utilisé avec un champ de mesure resserré pour la détermination de profils, dépendants du lieu, de valeurs de masse/humidité, **caractérisé en ce que** le contenu (2) de la capsule (1) est déplacé par accélération ou force de gravité sur un côté de la cavité de capsule et **en ce que** toutes les capsules (1) sont déplacées avec la même vitesse à travers le volume de mesure (12) et la masse/humidité globale des capsules (1) est déterminée par intégration.

6. Procédé selon l'une quelconque des revendications 1, 4 ou 5, **caractérisé en ce que** l'humidité de la capsule (1) est déterminée à l'aide de micro-ondes en fonction du point de mesure par rapport à la longueur de la capsule.

7. Procédé selon l'une quelconque des revendications 1 à 6, **caractérisé en ce que** les mesures sont effectuées avec une fréquence d'hyperfréquence de micro-ondes de 1 à 50 GHz, en particulier de 1 à 5 GHz.

8. Dispositif pour mesurer la masse et/ou l'humidité du contenu de capsules (1) contenant en particulier des produits médicaux, pharmaceutiques à base de vitamines et des produits similaires, à l'aide de micro-ondes, avec un premier résonateur à hyperfréquence (3) avec un champ de mesure homogène par rapport à l'extension de la capsule (1), **caractérisé en ce qu'**il présente des dispositifs pour le transfert du contenu (2) de la capsule (1) par accélération ou force de gravité sur un côté de la cavité de capsule et un second résonateur à hyperfréquence (4) pour mesurer des profils de masse et/ou d'humidité de la capsule avec un champ de mesure étroit par rapport à la longueur de capsule.

9.  Dispositif pour mesurer la masse et/ou l'humidité du contenu de capsules (1) contenant en particulier des produits médicaux, pharmaceutiques à base de vitamines et des produits similaires à l'aide de micro-ondes, avec un premier résonateur à hyperfréquence (3) avec un champ de mesure homogène sur l'extension de la capsule (1), **caractérisé en ce qu'**il comprend un second résonateur à hyperfréquence (4) qui présente un volume de mesure limité de façon si stricte que de ce fait seule l'enveloppe de capsule peut être détectée.

10.  Dispositif pour mesurer la masse et/ou l'humidité du contenu de capsules (1) contenant en particulier des produits médicaux, pharmaceutiques à base de vitamines et des produits similaires à l'aide de micro-ondes, comprenant un résonateur à hyperfréquence (3) avec un champ de mesure homogène sur l'extension de la capsule (1), **caractérisé en ce qu'**il comprend des dispositifs de mesure infrarouge (8, 9) pour la mesure de l'humidité de l'enveloppe de capsule.

11.  Dispositif pour mesurer la masse et/ou l'humidité du contenu de capsules (1) contenant en particulier des produits médicaux, pharmaceutiques à base de vitamines et des produits similaires à l'aide de micro-ondes, comprenant un résonateur à hyperfréquence (4) avec un champ de mesure étroit par rapport à la longueur de capsule, **caractérisé en ce qu'**il présente des dispositifs pour le transfert du contenu (2) de la capsule (1) par accélération ou force de gravité sur un côté de la cavité de capsule et un dispositif (10, 11) pour mesurer la vitesse des capsules (1).

12.  Dispositif pour mesurer la masse et/ou l'humidité du contenu de capsules (1) contenant en particulier des produits médicaux, pharmaceutiques à base de vitamineset des produits similaires à l'aide de micro-ondes, comprenant un premier résonateur à hyperfréquence (4) avec un champ de mesure étroit par rapport à la longueur de capsule, **caractérisé en ce qu'**il présente des dispositifs pour transférer le contenu (2) de la capsule (1) par accélération ou force de gravité sur un côté de la cavité de capsule et un dispositif (12) pour transporter les capsules (1) avec une vitesse uniforme.

13.  Dispositif selon l'une quelconque des revendications 8 à 12, **caractérisé en ce qu'**il peut être exploité avec des fréquences d'hyperfréquence de 1 à 50 GHz, en particulier de 1 à 5 GHz.

Fig. 1

Fig. 2

Fig. 3

a)

b)

Fig. 4

EP 1 669 755 B1

Fig. 5

Fig. 6

16

Fig. 7

Fig. 8